# EUROPEAN PATENT APPLICATION

(11) **EP 4 851 752 A1**
(43) Date of publication of application: **09.12.2026**
(21) Application number: 25748432.9
(22) Date of filing: 09.01.2025
(51) Int. Cl.: G06F 3/01, A61B 5/26, A61B 5/397

(54) **VIBRATION DEVICE AND VIBRATION SYSTEM**

(30) Priority: 30.01.2024 JP 2024012103
(71) Applicant: Kyocera Corporation, Kyoto-shi, Kyoto 612-8501 (JP)
(72) Inventor: SUZUKI Takashi, Kyoto-shi, Kyoto 612-8501 (JP)
(74) Representative: Viering, Jentschura & Partner mbB Patent- und Rechtsanwälte
(86) International application number: PCT/JP2025/000532
(87) International publication number: WO 2025/164250

(57) **Abstract**

A vibration apparatus comprises a vibrator. The vibrator is capable of applying vibration to a specific location of a user. The vibrator vibrates at a first frequency in response to muscle at the specific location undergoing concentric contraction. The vibrator vibrates at a second frequency in response to the muscle at the specific location undergoing isometric contraction or eccentric contraction. The first frequency is the same as or higher than the second frequency.

## Description

### CROSS-REFERENCE TO RELATED APPLICATIONS

This application claims priority to Japanese Patent Application No. 2024-012103 filed in Japan on January 30, 2024, the entire disclosure of which is incorporated herein by reference.

### TECHNICAL FIELD

The present disclosure relates to a vibration apparatus and a vibration system.

### BACKGROUND OF INVENTION

Conventionally, technology related to improvement of sensory sensitivity utilizing a stochastic resonance phenomenon is known. Hereinafter, sensory sensitivity includes tactile sensitivity and somatosensory sensitivity. For example, Patent Literature 1 discloses a method in which a vibration pad that provides weak vibration is attached to an upper surface of a brake pedal to improve sensitivity of tactile sensation of a driver's sole in response to deceleration of a vehicle, thereby enabling improvement of brake operation compared to conventional methods.

### CITATION LIST

### PATENT LITERATURE

Patent Literature 1: Japanese Patent Application Laid-Open No. 2017-138764

### SUMMARY

A vibration apparatus according to a first aspect comprises:
a vibrator capable of applying vibration to a specific location of a user, configured to vibrate at a first frequency in response to muscle at the specific location undergoing concentric contraction, and configured to vibrate at a second frequency in response to the muscle at the specific location undergoing isometric contraction or eccentric contraction, wherein
the first frequency is the same as the second frequency or is higher than the second frequency.

A vibration system according to a second aspect comprises:
a vibrator capable of applying vibration to a specific location of a user; and
an information processing unit configured to vibrate the vibrator at a first frequency in response to muscle at the specific location undergoing concentric contraction, and configured to vibrate the vibrator at a second frequency in response to the muscle at the specific location undergoing isometric contraction or eccentric contraction.

### BRIEF DESCRIPTION OF THE DRAWINGS

[FIG. 1] is an external view of a vibration apparatus according to a first embodiment of the present disclosure.
[FIG. 2] is an external view of a modified example of the vibration apparatus of FIG. 1.
[FIG. 3] is a functional block diagram showing a configuration of a control device of FIG. 1.
[FIG. 4] is a flowchart for explaining vibration processing executed by an information processing unit of FIG. 3.
[FIG. 5] is an external view of a vibration apparatus according to a second embodiment of the present disclosure.
[FIG. 6] is a functional block diagram showing a configuration of a control device of FIG. 5.
[FIG. 7] is a flowchart for explaining vibration processing executed by an information processing unit of FIG. 6.
[FIG. 8] is an external view of a vibration apparatus for experimentation.
[FIG. 9] is a graph displaying a target line and an actual line in a motor function confirmation test.
[FIG. 10] is a graph showing a first relative error, a second relative error, and a third relative error for each specific frequency.
[FIG. 11] is a graph plotting, by age, frequencies at which relative error is minimized in a first section and a third section for a plurality of subjects.

### DESCRIPTION OF EMBODIMENTS

Conventionally, a stochastic resonance phenomenon is known in which, in a nonlinear system such as a bistable system and a threshold system, by adding weak noise below a threshold to a signal, a probability that the signal exceeds the threshold and becomes stronger increases, and an ability to detect a weak signal is enhanced. As an example, it is known that tactile sensitivity of a part of a human body including a finger, a hand, and a foot is improved by applying weak vibration to an extent that a user does not feel. It is also known that improvement of tactile sensitivity causes improvement of motor function that performs feedback control using tactile information.

According to the method described in Patent Literature 1, tactile sensitivity of a driver is improved by weak vibration. This is a fact known as a stochastic resonance phenomenon. However, Patent Literature 1 does not disclose a method for effectively causing a stochastic resonance phenomenon to occur in accordance with a state of muscle that performs motor function. The present disclosure relates to improvement of motor function based on sensory sensitivity utilizing a stochastic resonance phenomenon.

Hereinafter, embodiments of the present disclosure will be described with reference to the drawings. In components shown in the following drawings, the same reference signs are assigned to the same components.

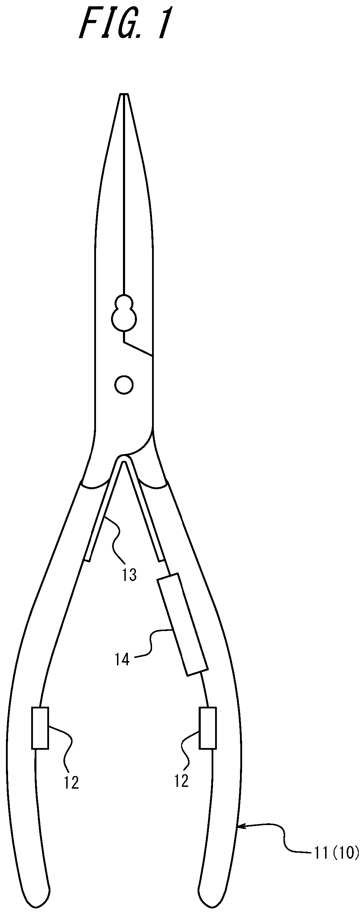

As shown in FIG. 1, a vibration apparatus 10 according to a first embodiment of the present disclosure may be embodied, for example, as an instrument 11 (hereinafter also referred to as a "first instrument") that is gripped such that an opening/closing degree is adjustable. The first instrument 11 may be, for example, a gripping tool such as a radio pliers, pliers, or nippers, scissors, or tweezers.

The vibration apparatus 10 is configured to include a vibrator 12. The vibration apparatus 10 may further includes a detector 13 and a control device 14.

The vibrator 12 is capable of applying vibration to a specific location of a user. In the first instrument 11, the specific location of the user is, for example, an inner surface side from an interphalangeal joint to a tip of a thumb, and an inner surface side from a proximal interphalangeal joint to a tip of an index finger. The inner surface side may mean a flexion side of a finger. Structurally, the vibrator 12 may be disposed on a gripping surface of the first instrument 11 that faces the user's fingers when the instrument is gripped. For example, the vibrator 12 may be capable of applying vibration by being provided in a vicinity of a location where contact with the user is expected in the vibration apparatus 10. In the first instrument 11, the vibrator 12 may be provided on an inner side or an outer side of a vicinity of a center in a longitudinal direction of a handle.

The vibrator 12 may be capable of vibrating at different frequencies. The vibrator 12 vibrates, for example, at a first frequency and a second frequency. The vibrator 12 may vibrate at the first frequency and the second frequency based on control by the control device 14 as described later. A vibration intensity at which the vibrator 12 vibrates at the first frequency and the second frequency may be below a threshold of human body sensation. Therefore, the vibrator 12 may apply vibration that is not perceived by the user to the specific location of the user.

The vibrator 12 may be any vibrator that generates weak vibration. The vibrator is, for example, a piezo element or a linear vibration actuator. The vibrator may be configured by using at least one element of one type, or may be configured by using a plurality of elements of different types.

The detector 13 may detect a contraction state of muscle at the specific location of the user. The detector 13 may directly detect the contraction state of the muscle, or may indirectly detect the contraction state. In the first embodiment, the detector 13 indirectly detects the contraction state of the muscle at the specific location.

In the first embodiment, the detector 13 may specifically be a bending sensor. The detector 13, which is a bending sensor, may be provided at a fulcrum of the first instrument 11. The detector 13, which is a bending sensor, may detect the opening/closing degree of the first instrument 11. As described later, the detector 13, which is a bending sensor, causes detection of the contraction state of the muscle at the specific location of the user based on temporally continuous detection results of the opening/closing degree.

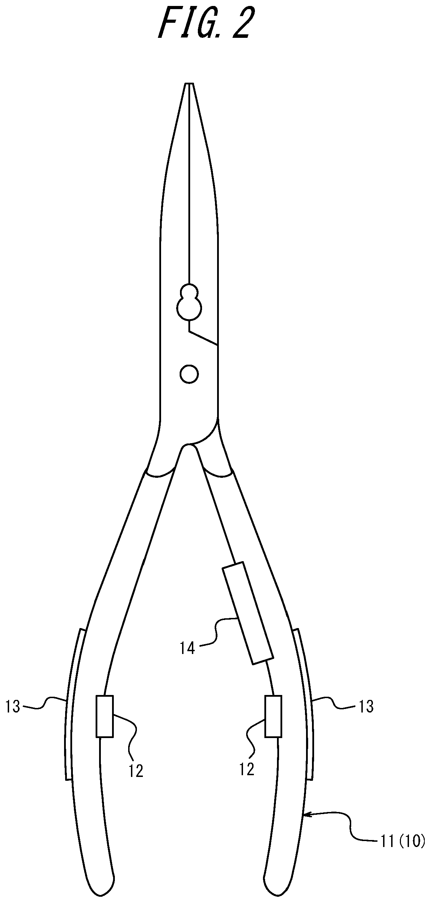

Alternatively, in the first embodiment, as shown in FIG. 2, the detector 13 may specifically be a pressure sensor. The detector 13, which is a pressure sensor, may be provided on an outer side of a handle of the first instrument 11. As described later, the detector 13, which is a pressure sensor, causes detection of the contraction state of the muscle at the specific location of the user based on temporally continuous detection results of pressure.

Alternatively, in the first embodiment, the detector 13 may specifically be a temperature sensor. The detector 13, which is a temperature sensor, may be provided on an outer side of a handle of the first instrument 11. As described later, the detector 13, which is a temperature sensor, causes detection of the contraction state of the muscle at the specific location of the user based on temporally continuous detection results of temperature.

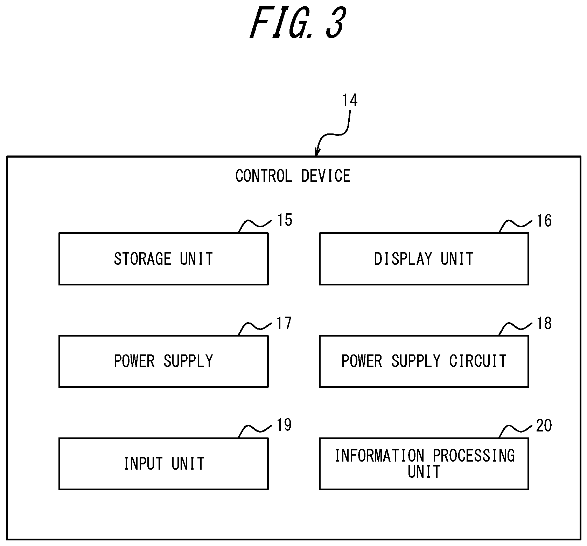

As shown in FIG. 3, the control device 14 may include, for example, a memory 15, a power supply 17, a power supply circuit 18, an input unit 19, and an information processing unit 20. The control device 14 may further include a display unit 16.

The memory 15 includes any storage device such as, for example, RAM (Random Access Memory) and ROM (Read Only Memory). The memory 15 may store various programs that cause the information processing unit 20 to function, and various information used by the information processing unit 20.

The power supply 17 may supply electric power for driving the control device 14. The power supply 17 is, for example, a battery. The power supply circuit 18 may adjust electric power supplied by the power supply 17 to a current value and a voltage value for driving components of the control device 14 and output the adjusted electric power. Electric power may be supplied to the vibrator 12 and the detector 13 via the power supply circuit 18.

The input unit 19 may detect an operation input by a user. The input unit 19 includes, for example, an input device such as a button. Alternatively, the input unit 19 may acquire an operation input by a user at an external device through communication.

The information processing unit 20 is configured to include at least one processor, at least one dedicated circuit, or a combination thereof. The processor is a general-purpose processor such as a CPU (Central Processing Unit) or a GPU (Graphics Processing Unit), or a dedicated processor specialized for specific processing. The dedicated circuit may be, for example, an FPGA (Field-Programmable Gate Array), an ASIC (Application Specific Integrated Circuit), or the like. The information processing unit 20 controls operation of the vibration apparatus 10.

The information processing unit 20 may acquire continuous detection results from the detector 13. The information processing unit 20 may estimate a contraction state of the muscle at the specific location of the user based on the continuous detection results.

In a configuration in which the detector 13 is a bending sensor, the information processing unit 20 may estimate that the muscle at the specific location is in an isometric contraction mode in response to the opening/closing degree being constant according to a detection result of the bending sensor. Further, the information processing unit 20 may estimate that the muscle at the specific location is in an eccentric contraction mode in response to the opening/closing degree changing in an opening direction according to the detection result of the bending sensor. Further, the information processing unit 20 may estimate that the muscle at the specific location is in a concentric contraction mode in response to the opening/closing degree changing in a closing direction according to the detection result of the bending sensor.

In a configuration in which the detector 13 is a pressure sensor, the information processing unit 20 may estimate that the muscle at the specific location is in an isometric contraction mode in response to pressure detected by the pressure sensor being substantially constant. Further, the information processing unit 20 may estimate that the muscle at the specific location is in an eccentric contraction mode in response to the pressure detected by the pressure sensor decreasing. Further, the information processing unit 20 may estimate that the muscle at the specific location is in a concentric contraction mode in response to the pressure detected by the pressure sensor increasing.

In a configuration in which the detector 13 is a temperature sensor, the information processing unit 20 may estimate that the muscle at the specific location is in an isometric contraction mode in response to temperature detected by the temperature sensor being substantially constant. Further, the information processing unit 20 may estimate that the muscle at the specific location is in an eccentric contraction mode in response to the temperature detected by the temperature sensor decreasing. Further, the information processing unit 20 may estimate that the muscle at the specific location is in a concentric contraction mode in response to the temperature detected by the temperature sensor increasing.

The information processing unit 20 vibrates the vibrator 12 at the first frequency in response to estimating that the muscle at the specific location of the user is undergoing concentric contraction. The information processing unit 20 vibrates the vibrator 12 at the second frequency in response to estimating that the muscle at the specific location of the user is undergoing isometric contraction or eccentric contraction. The information processing unit 20 may vibrate at different frequencies depending on whether the estimated state of the muscle is isometric contraction or eccentric contraction.

The first frequency is a frequency suitable for causing a stochastic resonance effect to occur in muscle undergoing concentric contraction. The first frequency is, for example, a frequency arbitrarily selected in a range of 300 to 500 Hz. The first frequency is preferably selected in a range of 350 to 450 Hz, more preferably selected in a range of 380 to 420 Hz, and most preferably 400 Hz. The second frequency is a frequency suitable for causing a stochastic resonance effect to occur in muscle undergoing isometric contraction or eccentric contraction. The second frequency may be the same as the first frequency or may be different. A frequency suitable for causing a stochastic resonance effect to occur in muscle undergoing isometric contraction or eccentric contraction varies based on characteristic information such as age of the user. Therefore, the information processing unit 20 may determine the second frequency based on the characteristic information of the user. The characteristic information is not limited to age, and may be any factor that affects occurrence of a stochastic resonance effect, such as height, weight, sex, body fat percentage, or blood type.

The information processing unit 20 may acquire the characteristic information of the user based on an operation input detected by the input unit 19. The information processing unit 20 may read the second frequency corresponding to the characteristic information of the user from the memory 15. Alternatively, the information processing unit 20 may read a predetermined second frequency from the memory 15 based on a target age for use of the first instrument 11.

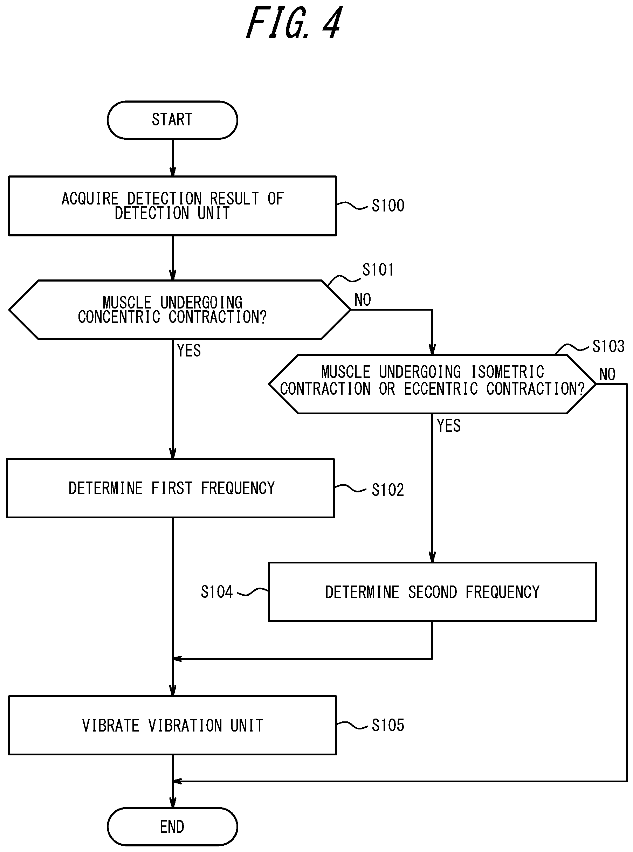

Next, vibration processing executed by the information processing unit 20 in the first embodiment will be described using a flowchart of FIG. 4. The vibration processing starts, for example, periodically.

In step S100, the information processing unit 20 acquires a detection result of the detector 13. After acquisition, the process proceeds to step S101.

In step S101, the information processing unit 20 determines whether or not the muscle is undergoing concentric contraction based on the detection result acquired in step S100 and a detection result acquired in the past. In response to the muscle undergoing concentric contraction, the process proceeds to step S102. In response to the muscle not undergoing concentric contraction, the process proceeds to step S103.

In step S102, the information processing unit 20 determines to vibrate at the first frequency. After determination, the process proceeds to step S105.

In step S103, the information processing unit 20 determines whether or not the muscle is undergoing isometric contraction or eccentric contraction based on the detection result acquired in step S100 and the detection result acquired in the past. In response to the muscle undergoing isometric contraction or eccentric contraction, the process proceeds to step S104. In response to the muscle undergoing neither isometric contraction nor eccentric contraction, the vibration processing ends.

In step S104, the information processing unit 20 determines to vibrate at the second frequency. After determination, the process proceeds to step S105.

In step S105, the information processing unit 20 controls the vibrator 12 to vibrate at the frequency determined in step S102 or step S104. After vibration, the vibration processing ends.

The vibration apparatus 10 of the first embodiment configured as described above comprises the vibrator 12 capable of applying vibration to a specific location of a user, configured to vibrate at the first frequency in response to estimating that muscle at the specific location is undergoing concentric contraction, and configured to vibrate at the second frequency in response to estimating that the muscle at the specific location is undergoing isometric contraction or eccentric contraction. With such a configuration, the vibration apparatus 10 can effectively cause a stochastic resonance phenomenon to occur in accordance with a state of muscle that performs motor function. Therefore, the vibration apparatus 10 can improve motor function based on sensory sensitivity of the user.

Further, in the vibration apparatus 10 of the first embodiment, the first frequency and the second frequency are below a threshold of human body sensation. With such a configuration, the vibration apparatus 10 can improve motor function based on sensory sensitivity without causing the user to feel discomfort.

Further, in the vibration apparatus 10 of the first embodiment, the first frequency is 400 Hz. With such a configuration, the vibration apparatus 10 can vibrate the vibrator 12 at a frequency suitable for causing a stochastic resonance effect to occur in muscle undergoing concentric contraction in response to the muscle undergoing concentric contraction.

Further, in the vibration apparatus 10 of the first embodiment, the second frequency is determined based on characteristic information of the user. With such a configuration, the vibration apparatus 10 can vibrate the vibrator 12 at a frequency suitable for causing a stochastic resonance effect to occur in muscle in the contraction state in response to the muscle undergoing isometric contraction or eccentric contraction.

Next, a vibration apparatus according to a second embodiment of the present disclosure will be described. In the second embodiment, an object in which the vibration apparatus is embodied is different from the first embodiment. Hereinafter, the second embodiment will be described focusing on differences from the first embodiment. Note that the same reference signs are assigned to parts having the same configuration as the first embodiment.

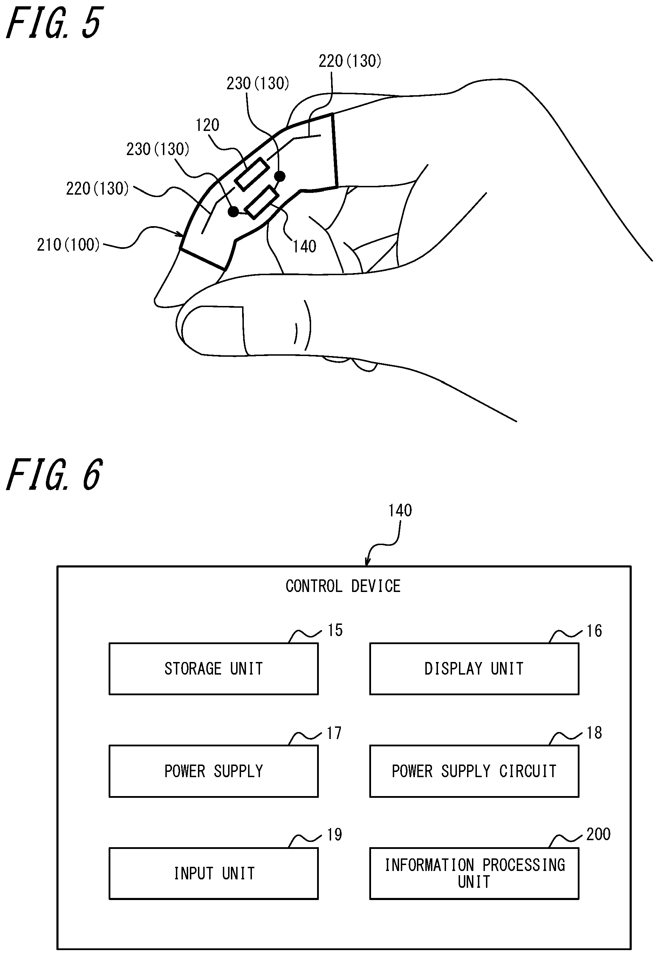

As shown in FIG. 5, a vibration apparatus 100 according to the second embodiment may be embodied, for example, as a wearing device 210 to be worn in a vicinity of a joint of a user. The wearing device 210 may be expected to be worn, for example, on any finger.

As in the first embodiment, the vibration apparatus 100 is configured to include a vibrator 120. Similar to the first embodiment, the vibration apparatus 100 may further be configured to include a detector 130 and a control device 140.

In the second embodiment, a configuration of the vibrator 120 other than an installation position is similar to the first embodiment. In the second embodiment, the vibrator 120 is capable of applying vibration to a specific location of a user, similar to the first embodiment. In the wearing device 210, the specific location of the user is, for example, an inner surface side from a metacarpophalangeal joint to a tip of any finger. Vibration to the specific location may be indirectly applied from a vicinity of the specific location. Therefore, for example, the vibrator 120 may be provided on an outer surface side of the finger in a state where the wearing device 210 is worn on the finger, and may apply vibration from the outer surface side toward the inner surface side. Alternatively, the vibrator 120 may be provided on an inner surface side of the finger in a state where the wearing device 210 is worn on the finger, and may directly apply vibration to the specific location.

As in the first embodiment, the detector 130 may detect a contraction state of muscle at the specific location of the user. In the second embodiment, the detector 130 may directly detect the contraction state of the muscle.

In the second embodiment, the detector 130 may specifically include a bending sensor 220 and a myoelectric measurement terminal 230.

The bending sensor 220 may be provided so as to be positioned in a vicinity of an interphalangeal joint and to be deformed along the joint in a state where the wearing device 210 is worn on a finger. In the example of FIG. 5, the bending sensor 220 is separately provided at both a distal interphalangeal joint and a proximal interphalangeal joint of an index finger. The bending sensor 220 causes detection of a contraction state of muscle at the specific location of the user based on temporally continuous detection results of a flexion state of the joint.

The myoelectric measurement terminal 230 may be provided so as to abut in a vicinity of the specific location of the user in a state where the wearing device 210 is worn on a finger. The myoelectric measurement terminal 230 causes detection of whether or not the specific location is contracting based on a detection result of myoelectric potential at an abutment location.

As shown in FIG. 6, the control device 140 may include, for example, a memory 15, a display unit 16, a power supply 17, a power supply circuit 18, an input unit 19, and an information processing unit 200. In the second embodiment, structures and functions of the memory 15, the display unit 16, the power supply 17, the power supply circuit 18, and the input unit 19 are the same as in the first embodiment. A structure of the information processing unit 200 is the same as in the first embodiment. In the second embodiment, functions other than a method of estimating a contraction state of muscle at the specific location by the information processing unit 200 are the same as in the first embodiment.

The information processing unit 200 may acquire myoelectric potential at an abutment location of the myoelectric measurement terminal 230. The information processing unit 200 may determine whether or not the myoelectric potential exceeds a threshold. The information processing unit 200 may determine that the muscle at the specific location is in a contraction state in response to the myoelectric potential exceeding the threshold. The information processing unit 200 may determine that the muscle at the specific location is in a relaxation state in response to the myoelectric potential being equal to or less than the threshold.

In response to determining that the muscle at the specific location is in a contraction state, the information processing unit 200 may determine whether the muscle is undergoing concentric contraction, isometric contraction, or eccentric contraction based on a detection result of the bending sensor 220. The information processing unit 200 may estimate that the muscle at the specific location is in an isometric contraction mode in response to the opening/closing degree being constant according to the detection result of the bending sensor 220. Further, the information processing unit 200 may estimate that the muscle at the specific location is in an eccentric contraction mode in response to the opening/closing degree changing in an opening direction according to the detection result of the bending sensor 220. Further, the information processing unit 200 may estimate that the muscle at the specific location is in a concentric contraction mode in response to the opening/closing degree changing in a closing direction according to the detection result of the bending sensor 220.

In the second embodiment, as in the first embodiment, the information processing unit 200 vibrates the vibrator 12 at the first frequency in response to estimating that the muscle at the specific location of the user is undergoing concentric contraction. The information processing unit 20 vibrates the vibrator 12 at the second frequency in response to estimating that the muscle at the specific location of the user is undergoing isometric contraction or eccentric contraction.

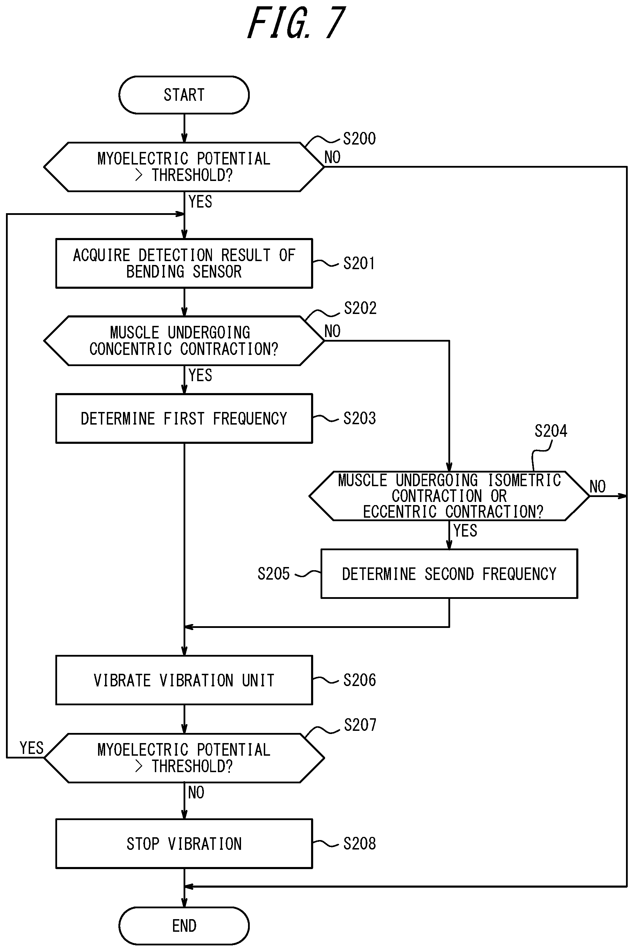

Next, vibration processing executed by the information processing unit 200 in the second embodiment will be described using a flowchart of FIG. 7. The vibration processing starts, for example, periodically.

In step S200, the information processing unit 200 determines whether or not a potential at an abutment location of the myoelectric measurement terminal 230 exceeds a threshold. In response to exceeding the threshold, the process proceeds to step S201. In response to not exceeding the threshold, the vibration processing ends.

In steps S201 to S206, the information processing unit 200 performs the same control as steps S100 to S105 of the vibration processing of the first embodiment. In step S206, after vibration, the process proceeds to step S207.

In step S207, the information processing unit 200 determines whether or not the potential at the abutment location of the myoelectric measurement terminal 230 exceeds the threshold. In response to exceeding the threshold, the process proceeds to step S201. In response to not exceeding the threshold, the process proceeds to step S208.

In step S208, the information processing unit 200 stops vibration of the vibrator 120. After stopping, the vibration processing ends.

The vibration apparatus 100 of the second embodiment configured as described above also comprises the vibrator 120 capable of applying vibration to a specific location of a user, configured to vibrate at the first frequency in response to estimating that muscle at the specific location is undergoing concentric contraction, and configured to vibrate at the second frequency in response to estimating that the muscle at the specific location is undergoing isometric contraction or eccentric contraction. Therefore, the vibration apparatus 100 can also improve motor function based on sensory sensitivity of the user.

Further, also in the vibration apparatus 100 of the second embodiment, the first frequency and the second frequency are below a threshold of human body sensation. With such a configuration, also in the vibration apparatus 100, motor function based on sensory sensitivity can be improved without causing the user to feel discomfort.

Further, also in the vibration apparatus 100 of the second embodiment, the first frequency is 400 Hz. With such a configuration, the vibration apparatus 100 can also vibrate the vibrator 120 at a frequency suitable for causing a stochastic resonance effect to occur in muscle undergoing concentric contraction in response to the muscle undergoing concentric contraction.

Further, also in the vibration apparatus 100 of the second embodiment, the second frequency is determined based on characteristic information of the user. With such a configuration, the vibration apparatus 100 can also vibrate the vibrator 120 at a frequency suitable for causing a stochastic resonance effect to occur in muscle in the contraction state in response to the muscle undergoing isometric contraction or eccentric contraction.

### EXAMPLES

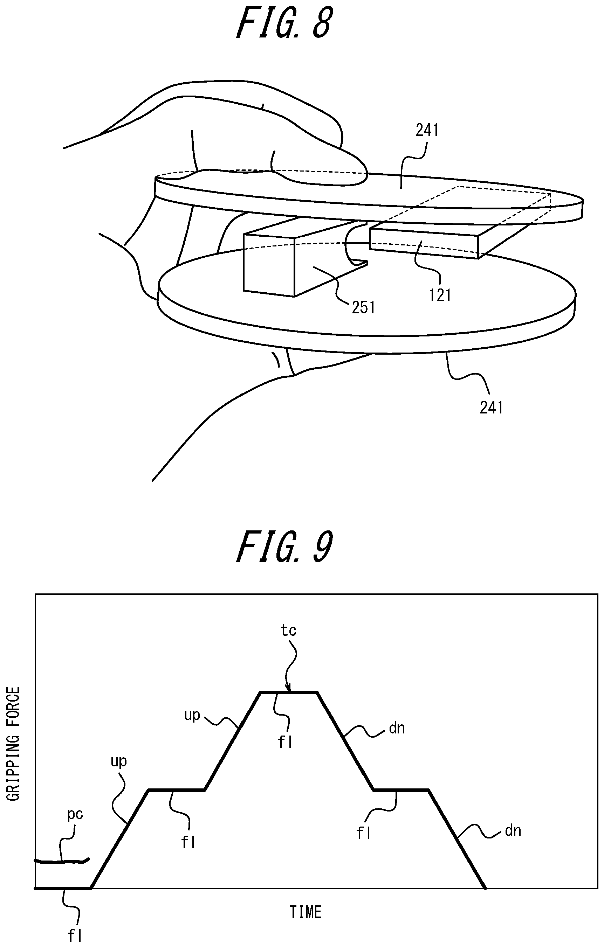

In order to show a frequency that effectively causes a stochastic resonance phenomenon to occur in accordance with a contraction state of muscle, a vibration experiment was performed using a vibration apparatus 101 for experimentation as shown in FIG. 8.

As shown in FIG. 8, the vibration apparatus 101 for experimentation is configured to include two circular plates 241, a load cell 251, and a vibrator 121. In the two circular plates 241, regions to be gripped are defined on surfaces opposite to surfaces facing each other. The two circular plates 241 are configured to be expandable and contractible between the regions to be gripped. Specifically, the two circular plates 241 are connected via a leaf spring bonded to a strain gauge of the load cell 251 and are expandable and contractible. The load cell 251 detects a gripping force when gripping the two circular plates 241. The vibrator 261 vibrates at a specific frequency adjusted arbitrarily.

A motor function confirmation test was performed using the vibration apparatus 101 for experimentation. In the motor function confirmation test, a subject was made to grip regions to be gripped of the vibration apparatus 101. As shown in FIG. 9, a target line tc in which a target value of a force for gripping the vibration apparatus 101 (hereinafter also referred to as "gripping force") was changed with elapsed time was displayed on a display. The target line tc included a first section fl in which the gripping force is constant, a second section up in which the gripping force is monotonically increased, and a third section dn in which the gripping force is monotonically decreased. On the display, an actual line pc depicting a change in an actual value of the gripping force detected by the load cell 251 with respect to time elapsed was displayed.

In a state where the vibrator 121 was vibrated at a specific frequency, an adjustment operation was performed in which the subject was made to adjust the gripping force with a goal of matching the actual line pc to the target line tc while showing the target line tc and the actual line pc to the subject. The adjustment operation was performed for each of a plurality of specific frequencies. The specific frequencies were measured as 0 Hz, white noise of 0 to 500 Hz, 100 Hz, 200 Hz, 250 Hz, 300 Hz, and 400 Hz. Note that a specific frequency of 0 Hz means no vibration.

During each adjustment operation, an average of |actual value - target value| (| represents absolute value) was calculated as an absolute error every 100 msec. An average value of absolute errors calculated in the first section fl during each adjustment operation was calculated as a first average value. An average value of absolute errors calculated in the second section up was calculated as a second average value. An average value of absolute errors calculated in the third section dn was calculated as a third average value.

A relative error for each frequency was calculated by dividing an average value of another frequency using the first average value, the second average value, and the third average value when the specific frequency is 0 Hz as reference values. For example, a first relative error when the specific frequency is 100 Hz is obtained by calculating (first average value at 100 Hz) / (first average value at 0 Hz). Similarly, a second relative error when the specific frequency is 100 Hz is obtained by calculating (second average value at 100 Hz) / (second average value at 0 Hz). Similarly, a third relative error when the specific frequency is 100 Hz is obtained by calculating (third average value at 100 Hz) / (third average value at 0 Hz). For other frequencies as well, calculation was performed using a calculation formula similar to the frequency of 100 Hz.

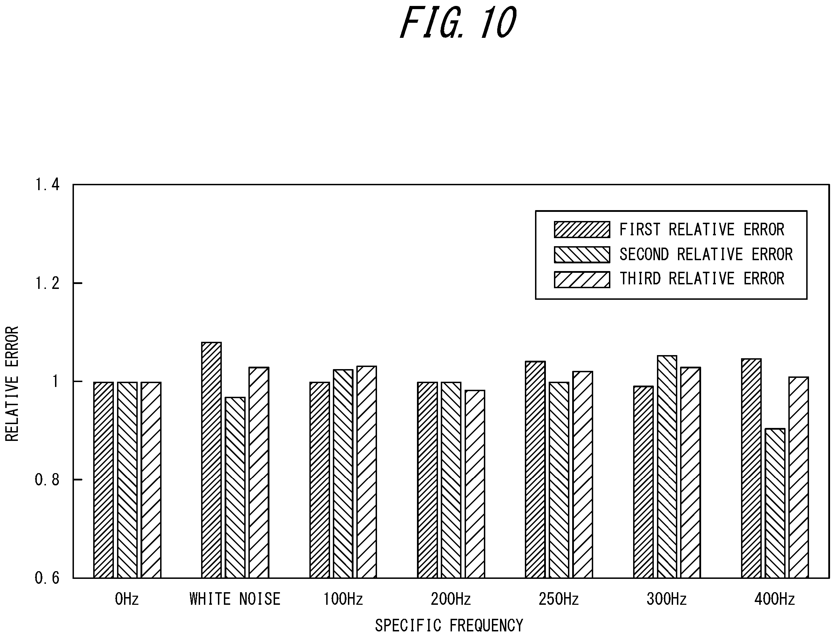

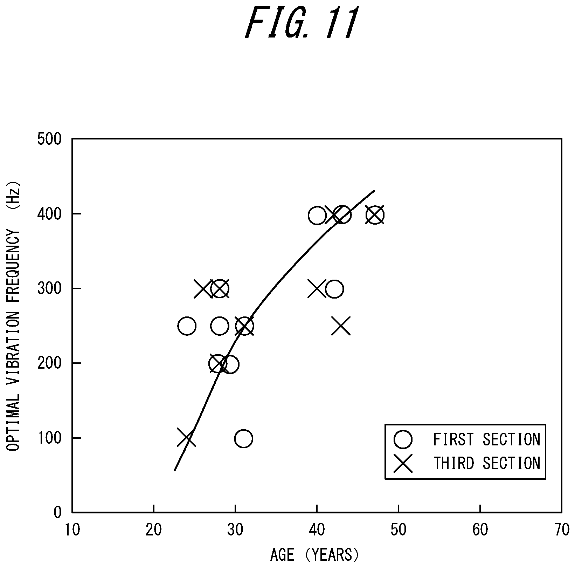

A plurality of subjects were made to perform the above adjustment operation, and a first relative error, a second relative error, and a third relative error were calculated for each specific frequency. The plurality of subjects included 14 subjects from their 20s to their 40s. An average value of relative errors of the plurality of subjects was calculated for each specific frequency. The calculation results are shown in FIG. 10. Further, for each of the plurality of subjects, frequencies at which the relative error is minimized in the first section fl and the third section dn were plotted by age, and an approximation curve was obtained. A graph plotting the frequencies is shown in FIG. 11 together with the approximation curve.

In one embodiment, (1) a vibration apparatus comprises:
a vibrator capable of applying vibration to a specific location of a user, configured to vibrate at a first frequency in response to muscle at the specific location undergoing concentric contraction, and configured to vibrate at a second frequency in response to the muscle at the specific location undergoing isometric contraction or eccentric contraction.

(2) In the vibration apparatus of (1) above,
a vibration intensity at which the vibrator vibrates at the first frequency and the second frequency is below a threshold of human body sensation.

(3) In the vibration apparatus of (1) or (2) above,
the first frequency is 400 Hz.

(4) In the vibration apparatus of any one of (1) to (3) above,
the second frequency is determined based on characteristic information of the user.

(5) In the vibration apparatus of (4) above,
the characteristic information is age of the user.

(6) In the vibration apparatus of any one of (1) to (5) above,
the vibration apparatus further comprises a detector configured to detect a contraction state of the muscle at the specific location.

(7) In the vibration apparatus of (6) above,
the detector includes a bending sensor.

(8) In the vibration apparatus of (7) above,
the bending sensor is provided at a fulcrum of an instrument to be gripped such that an opening/closing degree is adjustable, and is configured to detect the opening/closing degree.

(9) In the vibration apparatus of (7) above,
the bending sensor is configured to be deformed along a joint of the user adjacent to the specific location.

(10) In the vibration apparatus of (9) above,
the detector further includes a myoelectric measurement terminal for detecting myoelectric potential in a vicinity of the specific location.

In one embodiment, (11) a vibration system comprises:
a vibrator capable of applying vibration to a specific location of a user; and
an information processing unit configured to vibrate the vibrator at a first frequency in response to muscle at the specific location undergoing concentric contraction, and configured to vibrate the vibrator at a second frequency in response to the muscle at the specific location undergoing isometric contraction or eccentric contraction.

Although embodiments of the vibration apparatuses 10 and 100 have been described above, embodiments of the present disclosure may also take forms as a storage medium (for example, an optical disk, a magneto-optical disk, a CD-ROM, a CD-R, a CD-RW, a magnetic tape, a hard disk, or a memory card) on which a program is recorded, in addition to a method or a program for implementing the apparatus.

Further, an implementation form of a program is not limited to an application program such as object code compiled by a compiler or program code executed by an interpreter, and may be in a form of a program module incorporated in an operating system. Furthermore, the program may or may not be configured such that all processing is performed only in a CPU on a control board. The program may be configured such that a part or all of the program is performed by another processing unit mounted on an expansion board or an expansion unit added to the board as necessary.

Drawings for describing embodiments according to the present disclosure are schematic. Dimensional ratios and the like in the drawings do not necessarily match actual ones.

Although embodiments according to the present disclosure have been described based on various drawings and examples, it should be noted that a person skilled in the art can perform various modifications or alterations based on the present disclosure. Therefore, it should be noted that these modifications or alterations are included in a scope of the present disclosure. For example, functions and the like included in each component and the like can be rearranged so as not to be logically contradictory, and a plurality of components and the like can be combined into one or divided.

All constituent elements described in the present disclosure and/or all methods disclosed, or all steps of processing can be combined in any combination except for combinations in which these features are mutually exclusive. Further, each of features described in the present disclosure can be replaced with an alternative feature that serves an identical purpose, an equivalent purpose, or a similar purpose, unless explicitly denied. Therefore, unless explicitly denied, each of disclosed features is merely an example of a comprehensive series of identical or equivalent features.

Furthermore, embodiments according to the present disclosure are not limited to any specific configuration of the above-described embodiments. Embodiments according to the present disclosure can be extended to all novel features described in the present disclosure or combinations thereof, or all novel methods described, or steps of processing, or combinations thereof.

In the present disclosure, descriptions such as "first" and "second" are identifiers for distinguishing the configuration. Configurations distinguished by descriptions such as "first" and "second" in the present disclosure can exchange numbers in the configuration. For example, a first section can exchange identifiers "first" and "second" with a second section. Exchange of identifiers is performed simultaneously. The configuration is distinguished even after exchange of identifiers. Identifiers may be deleted. A configuration from which identifiers are deleted is distinguished by reference signs. Based only on descriptions of identifiers such as "first" and "second" in the present disclosure, interpretation of an order of the configuration or use as a basis for existence of an identifier with a smaller number should not be made.

### REFERENCE SIGNS

10, 100, 101 vibration apparatus
11 first instrument
12, 120 vibrator
13, 130 detector
14, 140 control device
15 memory
16 display unit
17 power supply
18 power supply circuit
19 input unit
20, 200 information processing unit
210 wearing device
220 bending sensor
230 myoelectric measurement terminal
241 circular plate
251 load cell
261 vibrator
dn third section
fl first section
up second section

## Claims

1. A vibration apparatus comprising a vibrator capable of applying vibration to a specific location of a user, configured to vibrate at a first frequency in response to muscle at the specific location undergoing concentric contraction, and configured to vibrate at a second frequency in response to the muscle at the specific location undergoing isometric contraction or eccentric contraction, wherein
the first frequency is the same as the second frequency or is higher than the second frequency.

2. The vibration apparatus according to Claim 1, wherein
a vibration intensity at which the vibrator vibrates at the first frequency and the second frequency is below a threshold of human body sensation.

3. The vibration apparatus according to Claim 1 or 2, wherein
the first frequency is 400 Hz.

4. The vibration apparatus according to any one of Claims 1 to 3, wherein
the second frequency is determined based on characteristic information of the user.

5. The vibration apparatus according to Claim 4, wherein
the characteristic information is age of the user.

6. The vibration apparatus according to any one of Claims 1 to 5, further comprising a detector configured to detect a contraction state of the muscle at the specific location.

7. The vibration apparatus according to Claim 6, wherein
the detector includes a bending sensor.

8. The vibration apparatus according to Claim 7, wherein
the bending sensor is provided at a fulcrum of an instrument to be gripped such that an opening/closing degree is adjustable, and is configured to detect the opening/closing degree.

9. The vibration apparatus according to Claim 7, wherein
the bending sensor is configured to be deformed along a joint of the user adjacent to the specific location.

10. The vibration apparatus according to Claim 9, wherein
the detector further includes a myoelectric measurement terminal for detecting myoelectric potential in a vicinity of the specific location.

11. A vibration system comprising:
a vibrator capable of applying vibration to a specific location of a user; and
an information processing unit configured to vibrate the vibrator at a first frequency in response to muscle at the specific location undergoing concentric contraction, and configured to vibrate the vibrator at a second frequency in response to the muscle at the specific location undergoing isometric contraction or eccentric contraction.
